# EUROPEAN PATENT SPECIFICATION

(11) **EP 1 418 887 B1**
(45) Date of publication and mention of the grant of the patent: **09.11.2005**
(21) Application number: 02746282.9
(22) Date of filing: 08.07.2002
(51) Int. Cl.: A61K 9/00, A61L 27/40, A61K 47/36

(54) **HEPARIN STENT**
HEPARIN-STENT
STENT A L'HEPARINE

(30) Priority: 27.07.2001 SE 0102621
(43) Date of publication of application: 19.05.2004
(73) Proprietor: Zoucas Kirurgkonsult AB, 21773 Malmö (SE)
(72) Inventor: HARNEK, Jan, S-217 73 Malmö (SE); ZOUKA, Eftichia-Vassiliki, S-217 73 Malmö (SE)
(74) Representative: Bjerre, Nils Birger Johannes
(86) International application number: PCT/SE2002/001356
(87) International publication number: WO 2003/011250

(56) References cited:
- EP-A2- 0 879 595
- WO-A1-95/07691
- SE-A- 0 000 363
- US-A- 5 529 986
- US-A- 5 591 227
- MYUNG HO JEONG ET AL.: 'Biological and genetic therapy for restenosis' CHONNAN J. MED. SCI. vol. 9, no. 1, 1996, pages 122 - 129, XP002957971

## Description

### Field of the Invention

The present invention relates to a medical device adapted for insertion into a human or animal body as well as a method for use thereof in promoting tissue healing and in treatment of restenosis and disorders related thereto.

### Background of the Invention

During the last years, local drug administration has become an increasingly more attractive means of treatment of various disorders. As is well known, local drug administration mainly offers both a reduced risk of unwanted systemic side-effects and much less general inconvenience for all parties involved. Hence, a vast number of various medical devices and methods providing direct application of drug(s) to a diseased site have been disclosed. Typical such medical devices and methods are disclosed in US 5 861 168, US 5 591 227, WO 96/35416, WO 99/08729 and EP 879 595.

Stenotic lesions of vasculature are common disorders which often lead to arterial occlusive disease. Indeed, the latter is the most frequently encountered problem of vascular disease, and particularly of cardiovascular disease. In general, approximately 50% of the patients with significant cardiovascular disease will be treated with percutaneous coronary angioplasty, whereby a balloon angioplasty is usually performed. However, the high incidence of restenosis, reaching 30-50% in several studies, following such ballon angioplasty continues to restrict the long-term success of this procedure (Kastrati, A., Schomig, A., Elezi, S., Schulen, H., Wilhelm, M., Dirschinger, J., Circ., 97, 2396 (1998)).

In order to treat the aforementioned resulting restenosis, stent implantation has lead to some success.

Various medical devices having a coating which provides local rapid release of nitric oxide (NO) have been disclosed as a potentially more successful alternative. Typical such medical devices are disclosed in WO 96/35416 referred to above. This reference suggests many types of medical devices providing release of NO, such as i) a medical device partially or completely coated with a nitric oxide adduct either as the coating per se or in a coating matrix, *ii)* a medical device partially or completely produced from a material which includes an NO adduct, and iii) a medical device derivatised with an NO adduct. As for coated stents, WO 96/35416 explicitly discloses only a Palmaz-Schatz stent coated with a layer of a bovine serum albumine (BSA) conjugate of S-nitrosothiol (Example 5). All the other examples relate to coated catheters. Related teachings are disclosed in WO 99/08729, where a balloon catheter coated with a layer of molsidomine is utilised.

Here it should be mentioned that molsidomine is a nitric oxide donor which belongs to the substance group of sydnonimines. This type of compounds are known for their ability to release NO without need of enzymatic catalysis (Lablanche, J-M. et al., *Circ*., 95 (1), 83 (1997)). Diethylenetriamine/nitric oxide adduct (DETA/NO) is a similar NO releasing compound. (Maragos C.M. et al. J. Med Chem. 34:3242-3247.(1991)

As for the aforementioned types of coated medical devices, two main problems are associated therewith. Firstly, the type of coating used is not potent enough to promote tissue healing, particularly vascular healing, to such an extent that beneficial long-term effects are attained. Accordingly, the hitherto known coatings are not potent enough to treat restenosis in such a manner that it ceases to be detrimental to the patient on a more long-term basis. Secondly, said type of coating elicits virtually no prophylactic effect. Furthermore, there is a general need for improving the biocompatibility of the surfaces of medical devices adapted for insertion into living tissue.

There is of course a strong demand in the art to provide a medical device which overcomes all of the above disadvantages.

EP 879 595 discloses a medical device having a coating comprising an internal reservoir.layer and an outer layer, where the outer layer comprises an ionic surfactant complexed to a biologically active material. The internal reservoir layer comprises a polymer incorporating the biologically active material. However, the present invention does not utilise any such ionic surfactant complex formation.

US 5 591 227 discloses stents coated with layers of polymer and fibrin incorporating a therapeutic substance. As set forth below, the content and design of those layers are substantially different from the present invention.

WO-A-0156646 is prior art according to A54(3) EPC The present invention differs from said NO application in that the biocompatible carrier is a sulphated glycosaminoglycan selected from a preferred group, see below. The above WO application has not been prepublished.

In summary, the characterising features of the medical device according to the present invention are neither disclosed nor suggested in any one of the aforementioned references.

### Disclosure of the Invention

According to the present invention, there is provided a novel medical device which overcomes the problems referred to above. Indeed, the features of the present medical device provide a solution of these problems also for many other types of disorders (*vide infra*) in addition to vascular damage(s) and restenosis. Owing to a careful choice of components, excellent biocompatibility is also provided. More specifically, the present invention relates to a medical device adapted for insertion into a human or animal body, characterised in that its exterior surface is coated with
*i)* an inner first layer of a biocompatible carrier comprising a sulphated glycosaminoglycan selected from heparin, heparan sulphate, dermatan sulphate and chondroitin sulphate including biocompatible fragments, derivatives and conjugates thereof and providing sustained release of a biologically active agent dissolved or dispersed therein;
*ii)* an outer second layer consisting of a film of said biologically active agent applied on said inner first layer, where said film optionally may contain at least one non-polymeric adjuvant, diluent or carrier.

As used herein, the expression "sulphated glycosaminoglycan" also encompasses molecules comprising a sulphated glycosaminoglycan moiety. Thus, said expression not only comprises the substances which are normally included, such as *e*.*g*. heparin, heparan sulphate, dermatan sulphate and chondroitin sulphate, but also biocompatible fragments, derivatives and conjugates of sulphated glycosaminoglycans.

Among the sulphated glycosaminoglycans, it is well known that *e*.*g*. heparin has excellent biocompatibility manifested in anti-coagulating properties and capacity of dissolving and preventing thromboses.

The expression "biologically active agent", as used herein, comprises any substance(s) which may yield a physiological response when administered to a living organism. Thus, said biologically active agent may also be an active metabolite, drug progenitor or a drug-conjugate, such as a drug-protein (*e.g*. drug-BSA) conjugate or a drug-spacer conjugate, where the protein or spacer is selected in such a manner that it will readily adhere to said inner first layer, *i*.*e*. to said biocompatible carrier. The conjugates may be formed by either covalent binding or other sufficiently strong intermolecular binding resulting from e*.g*. hydrophobic, hydrogen-binding or hydrophilic interactions.

It should be understood that said biologically active agent may also be a mixture of one or more physiologically active substances, which are used in a particular combination. In this case, the combination is present in both said first and second layer, albeit not necessarily in the same concentration and/or ratio.

The expression "sustained release", as used herein, means that said biocompatible carrier releases no more than 50-90 percent by weight (wt%) of said biologically active agent dissolved or dispersed therein within 7 days after insertion of said medical device into a human or animal body.

Preferably, said sulphated glycosaminoglycan is selected from heparin, heparan sulphate, dermatan sulphate and chondroitin sulphate, including biocompatible fragments, derivatives and conjugates thereof.

In one of the embodiments of the present invention, said sulphated glycosaminoglycan is heparin or a fragment thereof. It deserves mentioning that heparin is an endogenous sulphated mucopolysaccharide which occurrs naturally complex-bound to protein in various mammalian tissues, such as the intestine, liver and lung, and has then an average molecular weight (M_{w}) of up to 100 kDa. Commercially available preparations of heparin, e.g. as provided by Pharmacia Corp., typically have an M_{w} of between 6 and 20 kDa.

In the preferred embodiment, said sulphated glycosaminoglycan is a heparin conjugate. A conjugate with an organic polymer chain is preferable. The preparation of heparin conjugates, especially to an organic polymer chain, is well known in the art and disclosed inter alia in US 5 529 986, the teachings and citations of which are incorporated herein by reference.

Said organic polymer chain is typically selected from a polyaminoacid, preferably polylysine or polyornithine, polyamine, chitosan, polyimine, polyallylamine, a polysaccharide and an aliphatic polymer. Normally, the organic polymer chain is substantially straight-chained.

It is particularly preferred that said sulphated glycosaminoglycan is conjugated to said organic polymer chain via a coupling (spacer) moiety. The most suitable coupling moiety is provided via a heterobifunctional coupling reagent, preferably N-succinimidyl-3-(2-pyridyldithio)-propionate (SPDP).

It is most preferred that said sulphated glycosaminoglycan is a heparin conjugate having from about 30 to 500, preferably from about 100 to 250, heparin molecules conjugated to said organic polymer. Said organic polymer suitably has an average molecular weight of from about 50 to 500 kDa, preferably at least about 100 kDa. According to the present invention, the most suitable organic polymer is selected from polylysine, chitosan and polyallylamine, where polylysine is the very most suitable choice.

For many biologically active agents, a sulphated glycosaminoglycan as the main and/or only component of said biocompatible carrier will provide the desired sustained release of a biologically active agent dissolved or dispersed therein. However, some biologically active agents may require that the sulphated glycosaminoglycan is admixed with at least one other polymeric carrier, where the function of the latter is to aid in providing and tuning in the desired (*e.g.* linear) sustained release profile of said biologically active agent dissolved or dispersed in the resulting admixture. Should the need arise, such admixing is readily accomplished by a person skilled in the art. See *e.g*. US 4 767 628 and US 5 869 103, which disclose linear release of a biologically active agent from polymer mixtures.

Thus, in another embodiment, said biocompatible carrier comprises said sulphated glycosaminoglycan admixed with at least one polymeric carrier, where said polymeric carrier is not a sulphated glycosaminoglycan. Said polymeric carrier is preferably biodegradable and selected to aid in providing the desired sustained release profile of said biologically active agent dissolved or dispersed in said biocompatible carrier. The function of the polymeric carrier may also be to solubilise the sulphated glycosaminoglycan and the biologically active agent and/or to confer particular adhesive, mechanical or thermal properties.

Said polymeric carrier is preferably selected from poly fatty acid esters, polyurethane and other pharmaceutically acceptable polymeric carriers known in the art. Thus, the following polymers can provide a suitable biocompatible carrier according to the present invention: poly fatty acid esters [*e.g.* homopolymer (*e.g*. polylactic acid) of fatty acid or copolymer (*e.g*. copolymer of lactic acid/glycolic acid, copolymer of 2-hydroxy butyric acid/glycolic acid) of two or more fatty acids, a mixture of the homopolymer and/or copolymer *(e.g***.** a mixture of polylactic acid and copolymer of 2-hydroxybutyric acid/glycolic acid), examples of the fatty acid include α-hydroxycarboxylic acid *(e.g**.*** glycolic acid, lactic acid, 2-hydroxy butyric acid, 2-hydroxyvaleric acid, 2-hydroxy-3-methyl butyric acid, 2-hydroxycaproic acid, 2-hydroxyisocaproic acid, 2-hydroxycaprylic acid), cyclic dimers of α-hydroxycarboxylic acids (*e.g*. glycolide, lactide), hydroxydicarboxylic acid (*e.g*. malic acid), hydroxytricarboxylic acid (*e.g*. citric acid)], poly-α-cyanoacrylate, polyalkylene oxalates (*e.g.* polytrimethylene oxalate, polytetramethylene oxalate), poly ortho esters, poly ortho carbonates and other polycarbonates (*e.g*. polyethylene carbonate, poly-ethylenepropylene carbonate), polyamino acids (*e.g*. poly-γ-benzyl-L-glutamic acid, poly-L-alanine, poly-γ-methyl-L-glutamic acid), polylysine, and the like. Further examples of a suitable biocompatible carrier include polyacrylic acid, polymethacrylic acid, copolymer of acrylic acid and methacrylic acid, polyethyle glycol, silicon polymer, dextran stearate, ethylcellulose, acetylcellulose, maleic anhydride copolymers, ethylene-vinylacetate copolymer, polyvinyl acetate, polyvinyl alcohol, polyacrylamide and the like. These polymers may be used alone or in combination. They may be used in the form of a copolymer or mere mixture of these two or more polymers. They may be in the form of salts thereof. The affinity for the adsorbed molecular coating *e.g*. film can be enhanced by attachment of phenylboronic acid moities. For the purposes of the present invention, D-, L- and D,L-isomers are equally suitable.

Preferably, said non-polymeric adjuvant, diluent or carrier is selected from phosphorylcholine and derivatised phosphorylcholine, ionic or non-ionic surfactants, buffer salts, albumines, liposomes, and contrast medium; preferably iohexole.

As a non-limiting example of suitable derivatised phosphorylcholines, mention can be made of the compounds disclosed in WO 91/13639 and WO 93/22320, the entire teachings of which are incorporated herein by reference.

Moreover, it is preferred that said poly fatty acid ester and polyurethane has an average molecular weight in the range of from about 5 kDa to 200 kDa, preferably from about 10 to 100 kDa.

As an example of a heparin conjugate useful in the present invention mention can be made of the JOMED Heparin Surface material employed in the commercially available stent JOSTENT®Flex (manufactured by JOMED GmbH, Rangendingen, DE). This heparin conjugate has a M_{w} in the order of about 1000 kDa.

Preferably, said poly fatty acid ester is polylactic acid (PLA), polyglycolic acid (PGA) or a copolymer of lactic acid and glycolic acid (PLGA). PLGA is particularly preferred, as it is also commercially available in many varieties (*e.g*. RG756, RG502H and RG504H provided by Boehringer Ingelheim, DE). Other preferred polymers are poly-α-cyanoacrylate and a copolymer of 2-hydroxybutyric acid and glycolic acid.

When PLGA is used, its monomer ratio is preferably about 100/0 to 50/50 (w/w). When a copolymer of 2-hydroxybutyric acid and glycolic acid is used, its monomer ratio is preferably about 100/0 to 25/75 (w/w).

The average molecular weight of PLGA and the copolymer of 2-hydroxybutyric acid and glycolic acid is preferably about 5 to 30 kDa. When a mixture of a polylactic acid (A) and a copolymer of 2-hydroxybutyric acid/glycolic acid (B) is used, the mixture can be used in a blend (w/w) ratio of about 10/90 to 90/10, preferably about 25/75 to 75/25.

The weight-average molecular weight of the polylactic acid (A) is preferably about 5 to 30 kDa.

The preferred proportion of glycolic acid in the copolymer (B) is about 40-70 mol%. The average molecular weight of the copolymer (B) is preferably about 5 to 25 kDa.

If desired, said biocompatible carrier may additionally contain other substances which are generally used in the preparation of pharmaceutical compositions. Typical such substances are pharmaceutically acceptable adjuvants, ionic or non-ionic surfactants, adhesives, stabilisers (often antioxidants), lubricants and pH regulators. All of these substances are well known in the art.

Said biologically active agent is preferably present in said inner and outer layer at a concentration of from 0.01 to 99 percent by weight (wt%). Preferably, said inner first layer has a thickness in the range of from about 0.1 to 1000 µm, preferably at least 0.5 µm.

In the present medical device, said biologically active agent could be an antiinflammatory drug, e.g. COX-1 and -2 inhibitors, prostaglandines, indomethacin, or diclofenac. However, said biologically active agent is preferably a compound capable of providing release of nitric oxide. It is further preferred that said compound is a diethylenetriamine/nitric oxide adduct (DETA/NO), sydnonimine or morpholino-sydnonimine. Said compound is preferably molsidomine or linsidomine.

One or more agents, *i.e.* adjuvants, which enhance the amount of NO delivered to the cells at the site to be treated can also be present. Such agents typically enhance the absorption of NO or its precursor, increase the activity of the NO-releasing compound and/or protect the NO-releasing compound from degradation. Particularly useful such agents are the vitamins B₆, B₁₂, C and E. Also useful in the practising of the present invention are folates, β-carotene, glutathione, coenzyme Q, cysteine, tocopherols, phenolic compounds, thiols, ubiquinones, dexomethasone, heparinoids, Ca²⁺-antagonists, nitrates, protein kinase inhibitors, anti-thrombin and antiproliferative agents, such as cytostatics, such as metotrexate, mitomycin C, doxyrubicin, somatostatin analogs, cytoschalasin B, rapamycin and cyclosporins.

In the present medical device, said exterior surface preferably consists of metal or a biocompatible organic or inorganic polymer. Said metal is preferably selected from gold, silver, platinum, stainless steel, titanium and biocompatible alloys thereof. Said biocompatible organic or inorganic polymer is preferably selected from fibrin, polytetrafluoroethylene (PTFE), silicone, silicone rubber, nylon and polyethylene perthalate (Dacron).

Moreover, it is preferred that said medical device is selected from catheters, guide wires, balloons, filters, vascular grafts, graft connectors, tubing, implants, sutures, surgical staples, stentgrafts and stents.

In the most preferred embodiment of the present invention, said medical device is a stent. Particularly preferred are JOSTENT®Flex and JOMED stentgrafts adapted for coronary use as well as JOSTENT®SelfX, JOSTENT®Peripheral and JOSTENT®Renal for peripheral use.

In addition, the present invention relates to a method for use of said medical device as set forth above. More specifically, the present invention further relates to a method for promoting tissue healing in a human or animal body, wherein said method comprises insertion of a medical device as set forth above into a site where tissue healing is required. Even more specifically, the present invention also relates to a method for treatment or prevention of restenosis and disorders related thereto in a human or animal body, wherein said method comprises insertion of a medical device as set forth above into a site where treatment or prevention of restenosis and disorders related thereto is required.

Said site is typically an artery, preferably a coronary artery, or a part of the gastrointestinal tract.

The above method is also applicable to the treatment or prevention of other disorders, such as inflammatory conditions or proliferative disorders, *e.g.* cancer diseases. A person skilled in the art will readily realise how to adapt, if necessary, the practising of the present method to the particular disorder and circumstances at hand.

As for the typical dosage of the biologically active agent, it varies within a wide range and depends on various factors, such as the particular requirements of each receiving indvidual and the particular medical device used. The required dosage range depends on the used agent and circumstance under which it is applied. The dosage is generally within the range of 0.001-100 mg/kg body weight, albeit also other ranges may be required under certain circumstances.

The present invention is further illustrated by the following non-limiting general experimental part.

The following figures depict the results obtained:

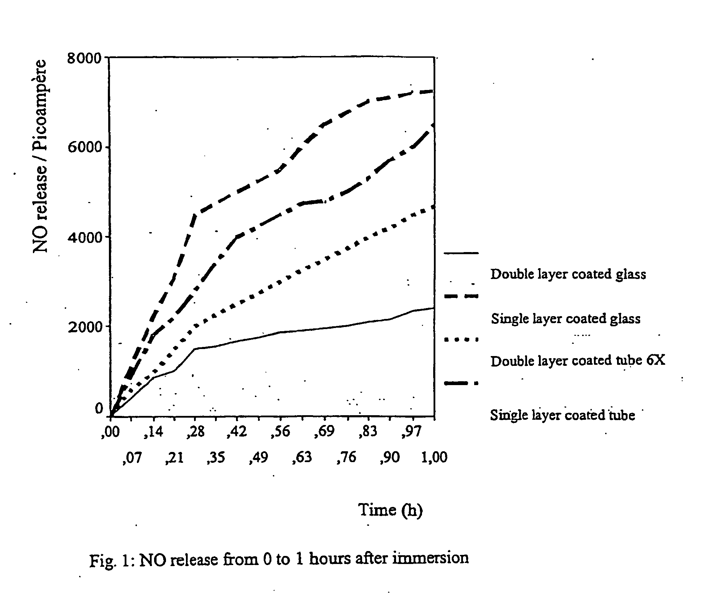

Fig. 1 shows in vitro release of nitric oxide into an aqueous medium, as measured by a conventional NO-electrode (pA registered), from glass beads and silastic tubing coated according to the invention. "Single layer coated" refers to a coating consisting of a film of 3-morpholino-sydnonimine (SIN-1) only, whereas "double layer coated" refers to a coating consisting of a heparin conjugate carrier layer into which SIN-1 has been absorbed, onto which layer a film of SIN-1 is present. The term "6X" means that six dipping/hardening cycles (see below) has been performed for the device in question. The release profile of nitric oxide for 1 h at room temperature is depicted in Fig. 1.

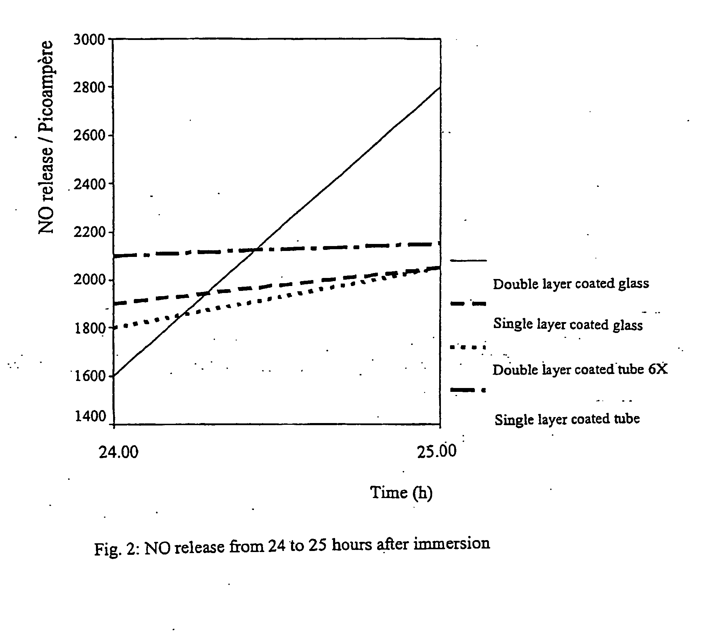

Fig. 2 shows the release profile of nitric oxide for the same coated glass beads and silastic tubing between 24 and 25 hours after continuous immersion into said aqueous medium.

### Example

Coating of a stent having a smooth stainless steel surface:

A JOSTENT®Flex stent made in electropolished Stainless steel 316L is coated by dipping it at room temperature into an aqueous, preferably paste-like, solution of heparin conjugate (polylysine conjugate with Heparin A as in Example 1 of US 5 529 986; M_{w} -3 300 kDa; heparin/polylysine ratio 240:1) as commercially available. A fluidizing solvent, such as ethanol, or an ethanol/water mixture may also be used. This layer (about 0.5 µm thick; several dipping/hardening cycles may be required) is sufficiently elastic to, after hardening, retain its structural integrity when the stent is subsequently expanded after insertion thereof into e.g. an artery. This heparin conjugate coated stent is then dipped into an aqueous 10⁻⁴ M solution of 3-morpholino-sydnonimine (SIN-1) for 5 min. A concentration as low as 10⁻⁸ M may provide the desired effect, and 1 nM may be effective. In this dipping step, the SIN-1 readily diffuses into and is absorbed throughout the heparin conjugate carrier. The applied coating is then allowed to harden by conventional means, *e.g.* by evaporation of the solvent at room temperature. Normally, both a first and a second layer are formed in this procedure (see the comments to the figures below). The first layer is heparin conjugate carrier incorporating SIN-1, whereas the second layer is a film of SIN-1 present on the heparin conjugate carrier layer.

The above procedure was also performed on glass beads and silastic tubes, and the release properties of these devices were investigated in vitro. The results are shown in the accompanying figures.

Fig. 1 shows that the immediate (acute) release of nitric oxide from the double layer coated devices according to the present invention is sufficiently rapid. Indeed, the "6X" coating (about 0.5 µm thick) of the silastic tubing displays a both rapid and nearly linear release profile for 1 h. The release of nitric oxide for 1 h is even more rapid from the devices coated with SIN-1 only ("single layer coated").

Fig. 2 shows that the single layer coated devices release virtually no or very little nitric oxide after 24 h. However, after this time the double layer coated devices according to the present invention still have a surprisingly substantial release rate of nitric oxide, and their release rates are now more rapid than those of the single layer coated devices.

After the hardening, if so desired, the thickness of the second layer can be increased further by dipping at 37°C the coated stent into an aqueous (or ethanol) solution containing SIN-1 in a concentration range of from about 10⁻⁸ M to 10⁻² M, preferably about 10⁻⁴ M. The solvent is then removed as above. Several such dipping/drying cycles may be performed for the the second layer as well.

Optionally, the aforementioned aqueous solution or paste of heparin conjugate may also contain dissolved SIN-1. In such a procedure, the second layer is applied after the hardening of the resulting heparin conjugate layer into which SIN-1 is incorporated.

The above procedure is readily applied on, or if necessary easily adapted to, virtually all of the commercially available stents. Typical such stents are Biodivysion™ (Biocompatibles Ltd., UK), BX high velocity Stainless Steel L316™ (Cordis, Johnson & Johnson Co., USA), NIR Primo Stainless Steel 316L™, NIRoyal Stainless Steel 316L™ (coated with a 7 µm layer of gold-plating), Radius self-expanding Nitinol™ stent (Medinol, Scimed, Boston Scientific Co., USA), S6™ and S7™ (AVE, Metronic, USA), Multilink Duett™ and Ultra™ (ACS, Guidant S.A., Belgium).

As further non-limiting examples of stents as well as guidewires and angioplasty balloons which are suitable in the practising of the present invention, mention can be made of those disclosed in "*Interventional Vascular Product Guide*". Ed.: Leon M.B., Mintz G.S., Publ. Martin Dunitz, 1999, and *"Endovascular Angioplasty material's catalog*", Europa Edition ISBN 2-913628-06-0, May 2001.

In short, the general potency of the present medical device is based primarily on the following principles. Firstly, upon insertion of the medical device into a body, a rapid release of the biologically active agent is provided. More specifically, the outer second layer will normally release at least 50% of its biologically active component within 1-24 h after insertion, thereby alleviating acute disorders. Secondly, the inner first layer will thereafter provide a sustained release (*vide supra*) of its biologically active component, thereby providing a long-term therapeutic effect as well as a prophylactic effect. This combined "pulsed" effect of the two layers provides a versatile treatment regimen, as substantiated by the results depicted in Fig. 1 and 2. The presence of the sulphated glycosaminoglycan confers maintained biocompatibility as well as prevents thromboses during the time of treatment.

Although the example above discloses the preparation of a coated stent only, it should be realised that the procedure is also readily adaptable for use on virtually any medical device. Hence, the features of the present medical device and method for use thereof are applicable within the field of medicine in general.

## Claims

1. Medical device adapted for insertion into a human or animal body, **characterised in that** its exterior surface is coated with
*i)* an inner first layer of a biocompatible carrier comprising a sulphated glycosaminoglycan selected from heparin, heparan sulphate, dermatan sulphate and chondroitin sulphate, including biocompatible fragments, derivatives and conjugates thereof and providing sustained release of a biologically active agent dissolved or dispersed therein;
*ii)* an outer second layer consisting of a film of said biologically active agent applied on said inner first layer, where said film optionally may contain at least one non-polymeric adjuvant, diluent or carrier.

2. Medical device according to claim 1, wherein said sulphated glycosaminoglycan is heparin or a fragment thereof.

3. Medical device according to claim 1, wherein said sulphated glycosaminoglycan is a heparin conjugate, preferably a conjugate with an organic polymer chain.

4. Medical device according to claim 3, wherein said organic polymer chain is selected from a polyaminoacid, preferably polylysine or polyornithine, polyamine, chitosan, polyimine, polyallylamine, a polysaccharide and an aliphatic polymer.

5. Medical device according to claim 4, wherein said organic polymer chain is substantially straight-chained.

6. Medical device according to any one of claims 3-5, wherein said sulphated glycosaminoglycan is conjugated to said organic polymer chain via a coupling moiety.

7. Medical device according to claim 6, wherein said coupling moiety is provided via a heterobifunctional coupling reagent, preferably *N*-succinimidyl-3-(2-pyridyldithio)-propionate (SPDP).

8. Medical device according to any one of claims 1 and 3-7, wherein said sulphated glycosaminoglycan is a heparin conjugate having from about 30 to 500, preferably from about 100 to 250, heparin molecules conjugated to said organic polymer.

9. Medical device according to claim 8, wherein said organic polymer has an average molecular weight of from about 50 to 500 kDa, preferably at least about 100 kDa.

10. Medical device according to claim 9, wherein said organic polymer is selected from polylysine, chitosan and polyallylamine.

11. Medical device according to any one of the preceding claims, wherein said biocompatible carrier comprises said sulphated glycosaminoglycan admixed with at least one polymeric carrier, where said polymeric carrier is not a sulphated glycosaminoglycan.

12. Medical device according to claim 11, wherein said polymeric carrier is selected from poly fatty acid esters and polyurethane.

13. Medical device according to claim 12, wherein said poly fatty acid ester or polyurethane has an average molecular weight in the range of from about 5 kDa to 200 kDa, preferably from about 10 to 100 kDa.

14. Medical device according to claim 13, wherein said poly fatty acid ester is polylactic acid (PLA), polyglycolic acid (PGA) or a copolymer of lactic acid and glycolic acid (PLGA).

15. Medical device according to any one of the preceding claims, wherein said non-polymeric adjuvant, diluent or carrier is selected from phosphorylcholine and derivatised phosphorylcholine, ionic or non-ionic surfactants, buffer salts, albumines, liposomes, and contrast medium; preferably iohexole.

16. Medical device according to any one of the preceding claims, wherein said biologically active agent is present in said inner and outer layer at a concentration of from 0.01 to 99 percent by weight.

17. Medical device according to any one of the preceding claims, wherein said inner first layer has a thickness in the range of from about 0.1 to 1000 µm, preferably at least 0.5 µm.

18. Medical device according to any one of the preceding claims, wherein said biologically active agent is a compound capable of providing release of nitric oxide.

19. Medical device according to claim 18, wherein said compound is a diethylenetrxamine/nitric oxide adduct or sydnonimine, preferably molsidomine or linsidomine.

20. Medical device according to any one of the preceding claims, wherein said exterior surface consists of metal or a biocompatible organic or inorganic polymer.

21. Medical device according to claim 20, wherein said metal is selected from gold, silver, platinum, stainless steel, titanium and biocompatible alloys thereof.

22. Medical device according to claim 20, wherein said biocompatible organic or inorganic polymer is selected.from fibrin, polytetrafluoroethylene (PTFE), silicone, silicone rubber, nylon and polyethylene perthalate (Dacron).

23. Medical device according to any one of the preceding claims, wherein said medical device is selected from catheters, guide wires, balloons, filters, vascular grafts, graft connectors, tubing, implants, suturs, surgical staples, heart valves, stentgrafts and stents.

24. Medical device according to claim 23, wherein said medical device is a stent.

## Patentansprüche

1. Medizinische Vorrichtung, die für das Einbringen in einen menschlichen oder tierischen Körper angepasst ist, **dadurch gekennzeichnet, dass** ihre Außenoberfläche beschichtet ist mit
i) einer inneren ersten Schicht einer biokompatiblen Trägersubstanz mit einem sulfatierten Glucosaminoglykan ausgewählt aus Heparin, Heparansulfat, Dermatansulfat und Chondroitinsulfat, einschließlich biokompatiblen Fragmenten, Derivaten und Konjugaten davon, und die eine fortwährende Abgabe eines darin gelösten oder dispergierten biologischen Wirkstoffs zur Verfügung stellt;
ii) einer äußeren zweiten Schicht, die aus einem Film des auf der inneren ersten Schicht aufgebrachten biologischen Wirkstoffs besteht, wobei der Film wahlweise wenigstens einen nicht polymeren Hilfsstoff, ein Verdünnungsmittel oder eine Trägersubstanz enthalten kann.

2. Medizinische Vorrichtung nach Anspruch 1, wobei das sulfatierte Glucosaminoglykan Heparin oder ein Fragment davon ist.

3. Medizinische Vorrichtung nach Anspruch 1, wobei das sulfatierte Glucosaminoglykan ein Heparinkonjugat ist, bevorzugt ein Konjugat mit einer organischen Polymerkette.

4. Medizinische Vorrichtung nach Anspruch 3, wobei die organische Polymerkette aus einer Polyaminosäure, bevorzugt Polylysin oder Polyornithin, Polyamin, Chitosan, Polyimin, Polyallylamin, einem Polysacharid und einem aliphatischen Polymer ausgewählt ist.

5. Medizinische Vorrichtung nach Anspruch 4, wobei die organische Polymerkette im Wesentlichen geradkettig ist.

6. Medizinische Vorrichtung nach einem der Ansprüche 3-5, wobei das sulfatierte Glucosaminoglykan mit der organische Polymerkette über einen Kopplungsrest verbunden ist.

7. Medizinische Vorrichtung nach Anspruch 6, wobei der Kopplungsrest über ein heterobifunktionales Kopplungsmittel, bevorzugt N-Succinimidyl-3-(2-pyridyldithio)-propionat (SPDP), zur Verfügung gestellt wird.

8. Medizinische Vorrichtung nach einem der Ansprüche 1 und 3-7, wobei das sulfatierte Glucosaminoglykan ein Heparinkonjugat mit etwa 30 bis 500, bevorzugt etwa 100 bis 250, mit dem organischen Polymer verbundenen Heparinmolekülen ist.

9. Medizinische Vorrichtung nach Anspruch 8, wobei das organische Polymer eine mittlere relative Molekülmasse von etwa 50 bis 500 kDa, bevorzugt wenigstens etwa 100 kDa, hat.

10. Medizinische Vorrichtung nach Anspruch 9, wobei das organische Polymer aus Polylysin, Chitosan und Polyallyamin ausgewählt ist.

11. Medizinische Vorrichtung nach einem der vorhergehenden Ansprüche, wobei die biokompatible Trägersubstanz das sulfatierte Glucosaminoglykan vermischt mit wenigstens einer polymeren Trägersubstanz umfasst, wobei die polymere Trägersubstanz kein sulfatiertes Glucosaminoglykan ist.

12. Medizinische Vorrichtung nach Anspruch 11, wobei die polymere Trägersubstanz aus Polyfettsäureestern und Polyurethan ausgewählt ist.

13. Medizinische Vorrichtung nach Anspruch 12, wobei der Polyfettsäureester oder das Polyurethan eine mittlere relative Molekülmasse im Bereich von etwa 5 kDa bis 200 kDa, bevorzugt von etwa 10 bis 100 kDa, hat.

14. Medizinische Vorrichtung nach Anspruch 13, wobei der Polyfettsäureester Polymilchsäure (PLA), Polyglykolsäure (PGA) oder ein Copolymer von Milchsäure und Glykolsäure (PLGA) ist.

15. Medizinische Vorrichtung nach einem der vorhergehendend Ansprüche, wobei der nichtpolymere Zusatzstoff, das Verdünnungsmittel oder die Trägersubstanz aus Phosphorylcholin und derivatisiertem Phosphorylcholin, ionischen oder nichtionischen oberflächenaktiven Stoffen, Puffersalzen, Albuminen, Liposomen und Kontrastmedium; bevorzugt Iohexol, ausgewählt ist.

16. Medizinische Vorrichtung nach einem der vorhergehenden Ansprüche, wobei der biologische Wirkstoff in der inneren und äußeren Schicht in einer Konzentration von 0,01 bis 99 Gewichtsprozent vorhanden ist.

17. Medizinische Vorrichtung nach einem der vorhergehenden Ansprüche, wobei die innere erste Schicht eine Dicke im Bereich von etwa 0,1 bis 1000 µm, bevorzugt wenigstens 0,5 µm, hat.

18. Medizinische Vorrichtung nach einem der vorhergehenden Ansprüche, wobei der biologische Wirkstoff eine Verbindung ist, die eine Abgabe von Stickstoffoxid zur Verfügung stellen kann.

19. Medizinische Vorrichtung nach Anspruch 18, wobei die Verbindung ein Diethylentriamin/Stickstoffoxid-Adukt oder Sydnonimin, bevorzugt Molsidomin oder Linsidomin, ist.

20. Medizinische Vorrichtung nach einem der vorhergehenden Ansprüche, wobei die Außenoberfläche aus Metall oder einem biokompatiblen organischen oder anorganischen Polymer besteht.

21. Medizinische Vorrichtung nach Anspruch 20, wobei das Metall aus Gold, Silber, Platin, rostfreiem Stahl, Titan und biokompatiblen Legierungen davon ausgewählt ist.

22. Medizinische Vorrichtung nach Anspruch 20, wobei das biokompatible organische oder anorganische Polymer ausgewählt wird aus Fibrin, Polytetrafluorethylen (PTFE), Silikon, Silikongummi, Nylon und Polyethylenperthalat (Dacron).

23. Medizinische Vorrichtung nach einem der vorhergehenden Ansprüche, wobei die medizinische Vorrichtung aus Kathetern, Führungsdrähten, Ballonen, Filtern, Gefäßprothesen, Prothesenverbindungsstücken, Schlauchmaterial, Implantaten, Nähten, chirurgischen Klammern, Herzklappen, Stentprothesen und Stents ausgewählt ist.

24. Medizinische Vorrichtung nach Anspruch 23, wobei die medizinische Vorrichtung ein Stent ist.

## Revendications

1. Dispositif médical adapté pour être inséré dans un corps humain ou animal, **caractérisé en ce que** sa surface extérieure est revêtue :
*i)* d'une première couche interne d'un vecteur biocompatible comprenant un glycosaminoglycanne sulfaté choisi parmi l'héparine, l'héparane sulfate, le dermatane sulfate et la chondroïtine sulfate, comprenant les fragments, dérivés et conjugués biocompatibles de ceux-ci et fournissant une libération prolongée d'un agent biologiquement actif dissous ou dispersé dans celui-ci ;
*ii*) d'une seconde couche externe constituée par un film dudit agent biologiquement actif appliqué sur ladite première couche interne, dans laquelle ledit film contient facultativement au moins un adjuvant, diluant ou vecteur non polymère.

2. Dispositif médical selon la revendication 1, dans lequel ledit glycosaminoglycanne sulfaté est l'héparine ou un fragment de celle-ci.

3. Dispositif médical selon la revendication 1, dans lequel ledit glycosaminoglycanne sulfaté est un conjugué d'héparine, de préférence un conjugué avec une chaîne polymère organique.

4. Dispositif médical selon la revendication 3, dans lequel ladite chaîne polymère organique est choisie parmi un acide polyaminé, de préférence la polylysine ou la polyornithine, la polyamine, le chitosan, la polyimine, la polyallylamine, un polysaccharide et un polymère aliphatique.

5. Dispositif médical selon la revendication 4, dans lequel ladite chaîne polymère organique est sensiblement linéaire.

6. Dispositif médical selon l'une quelconque des revendications 3 à 5, dans lequel ledit glycosaminoglycanne sulfaté est conjugué à ladite chaîne polymère organique via un groupe de couplage.

7. Dispositif médical selon la revendication 6, dans lequel ledit groupe de couplage est fourni via un réactif de couplage hétérobifonctionnel, de préférence le N-succinimidyl-3-(2-pyridyldithio)-propionate (SPDP).

8. Dispositif médical selon l'une quelconque des revendications 1 et 3 à 7, dans lequel ledit glycosaminoglycanne sulfaté est un conjugué d'héparine ayant d'environ 30 à 500, de préférence d'environ 100 à 250, molécules d'héparine conjuguées au dit polymère organique.

9. Dispositif médical selon la revendication 8, dans lequel ledit polymère organique a une masse moléculaire moyenne en poids comprise entre environ 50 et 500 kDa, de préférence d'au moins environ 100 kDa.

10. Dispositif médical selon la revendication 9, dans lequel ledit polymère organique est choisi parmi la polylysine, le chitosan et la polyallylamine.

11. Dispositif médical selon l'une quelconque des revendications précédentes, dans lequel ledit vecteur biocompatible comprend ledit glycosaminoglycanne sulfaté mélangé avec au moins un vecteur polymère, dans lequel ledit vecteur polymère n'est pas un glycosaminoglycanne sulfaté.

12. Dispositif médical selon la revendication 11, dans lequel ledit vecteur polymère est choisi parmi les esters des poly(acides gras) et le polyuréthane.

13. Dispositif médical selon la revendication 12, dans lequel ledit ester de poly(acide gras) ou ledit polyuréthane a une masse moléculaire moyenne dans la plage comprise entre environ 5 kDa et 200 kDa, de préférence entre environ 10 et 100 kDa.

14. Dispositif médical selon la revendication 13, dans lequel ledit ester de poly(acide gras) est l'acide polylactique (PLA), l'acide polyglycolique (PGA) ou un copolymère d'acide lactique et d'acide glycolique (PLGA).

15. Dispositif médical selon l'une quelconque des revendications précédentes, dans lequel ledit adjuvant, diluant ou vecteur non polymère est choisi parmi la phosphorylcholine et la phosphorylcholine dérivée, les surfactants ioniques ou non ioniques, les sels tampons, les albumines, des liposomes, et un milieu de contraste ; de préférence l'iohexole.

16. Dispositif médical selon l'une quelconque des revendications précédentes, dans lequel ledit agent biologiquement actif est présent dans lesdites couches interne et externe à une concentration comprise entre 0,01 et 99 % en poids.

17. Dispositif médical selon l'une quelconque des revendications précédentes, dans lequel ladite couche interne a une épaisseur dans la plage comprise entre environ 0,1 et 1000 µm, de préférence d'au moins 0,5 µm.

18. Dispositif médical selon l'une quelconque des revendications précédentes, dans lequel ledit agent biologiquement actif est un composé capable de libérer de l'oxyde nitrique.

19. Dispositif médical selon la revendication 18, dans lequel ledit composé est un adduit de diéthylènetriamine/oxyde nitrique ou la sydnonimine, de préférence la molsidomine ou la linsidomine.

20. Dispositif médical selon l'une quelconque des revendications précédentes, dans lequel ladite surface externe est constituée par un métal ou par un polymère biocompatible organique ou inorganique.

21. Dispositif médical selon la revendication 20, dans lequel ledit métal est choisi parmi l'or, l'argent, le platine, l'acier inoxydable, le titane et les alliages biocompatibles de ceux-ci.

22. Dispositif médical selon la revendication 20, dans lequel ledit polymère organique ou inorganique biocompatible est choisi parmi la fibrine, le polytétrafluoroéthylène (PTFE), la silicone, le caoutchouc silicone, le nylon et le perthalate de polyéthylène (Dracon).

23. Dispositif médical selon l'une quelconque des revendications précédentes, dans lequel ledit dispositif médical est choisi parmi les cathéters, les fils-guides, les ballonnets, les filtres, les greffes vasculaires, les raccords de greffon, les tubulures, les implants, les sutures, les agrafes chirurgicales, les valves cardiaques, les endoprothèses et les stents.

24. Dispositif médical selon la revendication 23, dans lequel ledit dispositif médical est un stent.
